# EUROPÄISCHE PATENTSCHRIFT

(11) **EP 3 523 650 B1**
(45) Veröffentlichungstag und Bekanntmachung des Hinweises auf die Patenterteilung: **18.03.2020**
(21) Anmeldenummer: 17784842.1
(22) Anmeldetag: 22.09.2017
(51) Int. Cl.: G01N 33/46, B23B 45/00, B25H 1/00, G01N 3/42

(54) **NADELFÜHRUNGSVORRICHTUNG FÜR EIN BOHRWIDERSTANDSMESSGERÄT, BOHRWIDERSTANDSMESSGERÄT SOWIE BOHRWIDERSTANDSMESSVERFAHREN ZUR BESCHAFFENHEITSUNTERSUCHUNG VON HOLZ**
NEEDLE-GUIDING DEVICE FOR A DRILLING RESISTANCE MEASUREMENT UNIT, DRILLING RESISTANCE MEASUREMENT UNIT, AND DRILLING RESISTANCE MEASUREMENT METHOD FOR INVESTIGATING THE NATURE OF WOOD
DISPOSITIF DE GUIDAGE DE MÈCHE POUR RÉSISTOGRAPHE, RÉSISTOGRAPHE ET PROCÉDÉ RÉSISTOGRAPHIQUE POUR ÉTUDIER LES CARACTÉRISTIQUES DU BOIS

(30) Priorität: 04.10.2016 DE 102016011776
(43) Veröffentlichungstag der Anmeldung: 14.08.2019
(73) Patentinhaber: IML Instrumenta Mechanik Labor GmbH, 69168 Wiesloch (DE)
(72) Erfinder: HUNGER, Erich, 76133 Karlsruhe (DE); HUNGER, Sebastian, 69181 Leimen (DE); HUNGER, Fabian, 69181 Leimen (DE)
(74) Vertreter: Mehl-Mikus, Claudia
(86) Internationale Anmeldenummer: PCT/EP2017/001132
(87) Internationale Veröffentlichungsnummer: WO 2018/065086

(56) Entgegenhaltungen:
- WO-A1-2012/167777
- AU-B2- 593 703
- DE-A1-102014 013 412
- DE-A1-102014 227 029
- DE-U1- 29 707 307
- GB-A- 1 103 810
- JP-A- 2003 103 409

## Beschreibung

Die Erfindung betrifft eine Nadelführungsvorrichtung, ein Bohrwiderstandsmessgerät mit einer erfindungsgemäßen Nadelführungsvorrichtung sowie ein Verfahren zur Beschaffenheitsuntersuchung von Holz mit dem Bohrwiderstandsmessgerät samt der Nadelführungsvorrichtung.

Aus dem Stand der Technik sind Bohrwiderstandsmessungen und Beschaffenheitsuntersuchungen bekannt, bei denen Bäume, Holzmasten oder Holz in anderer Form auf Defekte oder Fäulnis untersucht werden. Für diese Untersuchungen werden Bohrwiderstandsmessgeräte eingesetzt. Ein solches Bohrwiderstandsmessgerät ist beispielsweise aus DE 10 2013 001 711 bekannt.

In diesen Bohrwiderstandsmessgeräten werden für den überwachten Bohrwiderstandsmessvorgang dünne Bohrnadeln verwendet. Es ist bekannt, diese Bohrnadeln während dem Bohrvorgang gegen eine Bewegung quer zur Bohrrichtung abzustützen. So ist aus DE 100 31 395 ein Spezial-Becher-Teleskop bekannt, das die Bohrnadel in den Becherboden führt und einfahrbar ist, indem die kleineren Becher in den nächst größeren hineinfahren.

Eine Vorrichtung zur Holzprüfung ist auch aus DE 41 22 494 bekannt. Auch diese Vorrichtung weist zylindrische Teleskophülsen auf, in denen die Bohrnadel mittig geführt wird.

Aus DE 10 2010 018 249 A1 ist ein Handprüfgerät zur Beschaffenheitsuntersuchung von Holz mit einer Führungsvorrichtung zum geführten Einführen der Bohrnadel in das zu untersuchende Objekt bekannt. Die Führungsvorrichtung ist ein Teleskoprohr, das einen kürzeren inneren Rohrabschnitt umfasst, der in einem längeren äußeren Rohrabschnitt axial verschiebbar angeordnet ist und der an seinem Außenumfang eine längs angeordnete Messskala aufweist. Das Teleskoprohr ist über den inneren Rohrabschnitt auf die Antriebsvorrichtung nicht drehend aufgesetzt. Der innere und der äußere Rohrabschnitt weisen Führungsmittel zum zentral-axialen Führen der Bohrnadel auf, die sich zentralaxial von dem Bohrfutter durch den inneren Rohrabschnitt und durch den äußeren Rohrabschnitt erstreckt. So kann die Bohrnadel bei einem Einschieben des inneren Rohrabschnitts in den äußeren Rohrabschnitt durch die Führungsmittel geführt in das zu untersuchende Objekt eingetrieben werden.

DE 10 2013 015 131 B3 beschreibt ein Bohrfutter und eine Bohrnadel-Bohrfutter-Anordnung mit einer Klemmvorrichtung zur lösbaren Aufnahme der Bohrnadel. Weiter hat das Bohrfutter eine Frontabdeckung mit einer Öffnung für die Bohrnadel, wobei in der Öffnung eine Bohrnadel-Führungshülse einer Vorrichtung zur Führung der Bohrnadel drehbar gelagert ist, so dass die in der Bohrnadelführungsvorrichtung geführte Bohrnadel bei Kopplung des Bohrfutters mit der Antriebsvorrichtung co-zentrisch mit einer Motorabtriebswelle der Antriebsvorrichtung positioniert ist.

Eine weitere Vorrichtung zur Bohrwiderstandsmessung in einem zu untersuchenden Material ist aus DE 10 2014 227 029 A1 bekannt, die zur Führung der Bohrnadel mehrere entlang einer Bohrachse verschiebbare U-förmige Führungselemente mit unterschiedlich langen Schenkeln und einem die beiden Schenkel verbindenden Basisbereich aufweist. Dabei ist in jedem längeren Schenkel ein Durchgang für das Bohrwerkzeug zur Führung des Bohrwerkzeugs ausgebildet.

WO 2012/167777 A1 beschreibt eine Nadelwechselkartusche für ein Bohrwiderstandsmessgerät, die eine als Teleskoprohr mit einer Vielzahl teleskopierbarer Rohrabschnitte ausgebildete Führungsvorrichtung für eine Bohrnadel aufweist. Die Vielzahl der teleskopierbaren Rohrabschnitte erstreckt sich zwischen einem Arbeitsende, an dem eine Halterung zum drehfesten Halten der Bohrnadel angeordnet ist, und einem Andock-Ende, an dem eine Andock-Vorrichtung zur Kopplung des Teleskoprohrs mit einer Antriebsvorrichtung des Bohrmessgeräts angeordnet ist. Die Andockvorrichtung umfasst eine Kupplungsvorrichtung zur Drehmomentübertragung von der Antriebsvorrichtung des Bohrwiderstandsmessgeräts auf das Teleskoprohr und eine Getriebevorrichtung zur Übersetzung der Drehbewegung der Antriebsvorrichtung des Bohrmessgeräts in eine Linearbewegung des Teleskoprohrs.

GB 1,103,810 A beschreibt ein Erdbohrgerät zur Verwendung beim Pfostenaufstellen. Das Erdbohrgerät kann auf einem Rahmen montiert werden, der an einem selbstfahrenden Fahrzeug befestigt ist. Eine Doppelscherenspreize ist drehbar an einem Ende mit einem Untersetzungsantrieb und an dem anderen Ende, mittels eines Gegenlagers, mit einer Welle verbunden, die mit dem Antrieb in Eingriff gebracht und dadurch rotiert werden kann, und die mit einer Bohrspitze verbunden ist.

Bei der Verwendung der Teleskophülsen haben die aufeinander folgenden Teleskophülsen oder Becher eine große Kontaktfläche zur jeweils nächsten Teleskophülse. Durch die Bewegung der Teleskophülsen kommt es zu Reibung - diese Reibkräfte müssen überwunden werden. Der große Reibwert schlägt sich in der Vorschubmessung nieder.

Ausgehend von diesem Stand der Technik ist es Aufgabe der vorliegenden Erfindung, eine verbesserte Nadelführungsvorrichtung bereitzustellen.

Diese Aufgabe wird durch eine Nadelführungsvorrichtung mit den Merkmalen des Anspruchs 1 oder mit den Merkmalen des Anspruchs 8 gelöst.

Die weitere Aufgabe, ein verbessertes Bohrwiderstandsmessgerät mit einer verbesserten Nadelführung bereitzustellen, wird durch ein Bohrwiderstandsmessgerät mit den Merkmalen des unabhängigen Anspruchs 13 gelöst.

Die Aufgabe, ein verbessertes Verfahren zur Bohrwiderstandsmessung bereitzustellen, wird durch das Verfahren nach Anspruch 15 oder 16 gelöst.

Bevorzugte Ausführungsformen der Vorrichtungen oder des Verfahrens sind in den Unteransprüchen ausgeführt.

Eine erste Ausführungsform der erfindungsgemäßen Nadelführungsvorrichtung für ein vorbestimmtes Bohrwiderstandsmessgerät ist dazu ausgebildet, eine vorbestimmte Bohrnadel zu führen und mit einem Antrieb des Bohrwiderstandsmessgeräts operativ gekoppelt zu werden. Die Nadelführungsvorrichtung weist eine Abstützvorrichtung auf, die sich in Längsrichtung der vorbestimmten Bohrnadel erstreckt, und eine Nadelhalterung mit einem Bohrfutter, in das die Bohrnadel aufgenommen und eingespannt wird.

Eine "operative Kopplung" der Nadelführungsvorrichtung mit einem Bohrwiderstandsmessgerät bedeutet, dass sowohl eine kraftübertragende Verbindung von dem oder den Antrieben des Bohrwiderstandsmessgeräts zu den beweglichen Teilen der Nadelführungsvorrichtung als auch eine Datenübertragung zur Übertragung ermittelter Vorschub- und Widerstandsdaten hergestellt wird, soweit dies erforderlich ist.

"Vorbestimmte Bohrnadel" und "vorbestimmtes Bohrwiderstandsmessgerät" bedeutet, dass natürlich sowohl diese Nadel als auch die Maschine, die den Antrieb bereitstellt, aufeinander und auf das zu prüfende Holzobjekt abgestimmt sein müssen; und natürlich muss auch die Abstützvorrichtung für die Bohrnadel so dimensioniert sein, dass sie geeignet ist, einerseits die Länge der Nadel abzustützen und andererseits zuzulassen, dass die Nadel optimal genutzt werden kann.

Erfindungsgemäß ist nach einer ersten Ausführungsform die Abstützvorrichtung eine Scherengittervorrichtung, die ein Scherengitter aus Streben aufweist.

Das Scherengitter kann von einem ausgeklappten in einen eingeklappten Zustand überführt werden und spannt eine Ebene E auf. Zur Überführung in einen eingeklappten und in einen ausgeklappten Zustand liegen Gelenkkreuze vor, wie bei Scherengittern an sich bekannt. Es kreuzen sich jeweils zwei Streben, die mittels eines Gelenks miteinander verbunden sind. Erfindungsgemäß mündet nun aber jedes Gelenk in eine Stützstruktur, die sich normal zu der durch das Scherengitter aufgespannte Ebene E weg erstreckt. Vorliegend wird das Bauteil, das die Gelenkfunktion und die Stützfunktion aufweist, als Stützstruktur mit Gelenk bezeichnet. Jede Stützstruktur hat eine Durchtrittsöffnung, die als Aufnahme für die Bohrnadel dient. Die Durchtrittsöffnungen aller Stützstrukturen für die Bohrnadel fluchten miteinander und mit einer Öffnung der Nadelhalterung.

Durch die Führung der Bohrnadel in den Durchtrittsöffnungen kann die Nadel nahezu frei laufen und sich reibungsarm drehen. Um die Reibung weiter zu reduzieren, ist es möglich, das komplette Bauteil oder zumindest die Durchtrittsöffnungen mit speziellen Formen und Materialien zu gestalten, so dass die Reibung weiter reduziert wird. Die Durchtrittsöffnung kann etwa mit einer Auskleidung, bspw. aus Teflon, versehen werden.

Die Nadel wird durch das Scherengitter in regelmäßigen Abständen abgestützt, so dass sie auch bei Belastung vor Bruch geschützt ist, weil zwischen den Abstützpunkten eine bauchige Verformung der Nadel möglich ist und diese also ausweichen kann.

Die Streben des Scherengitters sind außer an den Gelenken an den Kreuzungspunkten auch noch an den Enden der Streben mit dem jeweils nächsten sich kreuzenden Strebenpaar verbunden, so dass nur an diesen Verbindungsstellen Reibung auftreten kann. Dabei können die Gelenke hinsichtlich Reibung optimiert sein, so dass eine kleinere Reibfläche entsteht und/oder diese Reibfläche wird mit einem Material versehen, das für kleinere Reibkräfte sorgt. Da die Störgröße Reibwiderstand auf die Bohrwiderstandsmessung, die den Vorschubwiderstand der Nadel erfassen, Einfluss nimmt, ist die Bohrwiderstandsmessung mit der erfindungsgemäßen Vorrichtung exakter als mit Vorrichtungen des Standes der Technik, weil diese Störgrößen minimiert wurden. Kurz: Die Kontaktfläche der Knotenpunkte ist wesentlich geringer als bei den Teleskophülsen, und je kleiner die Kontaktfläche, eine desto geringere Reibung tritt auf.

Das Scherengitter der Nadelführungsvorrichtung weist in ausgeklapptem Zustand eine Länge auf, die so gewählt ist, dass sich die vorbestimmte Bohrnadel in einer in dem Bohrfutter aufgenommenen Position nicht über das Scherengitter hinaus erstreckt. In eingeklapptem Zustand weist das Scherengitter eine Länge auf, die so ausgewählt ist, dass die vorbestimmte Bohrnadel mindestens zur Hälfte aus dem Scherengitter herausragt.

Bei der Anwendung, bei der die Bohrnadel des Bohrwiderstandsmessgeräts in einen Baum oder in ein anderes Holzobjekt (z. B. Leitungsmast) eingetrieben wird, führt die Nadelführungsvorrichtung die Bohrnadel, die in den Durchtrittsöffnungen der Stützstruktur läuft. Dabei ist das hintere Ende der Bohrnadel in einem Futter der Nadelhalterung oder des Bohrwiderstandsmessgeräts befestigt und das vordere Ende liegt am Holz an. Während des Bohrvorgangs wird das Scherengitter nach und nach zusammengeklappt und der aus dem Scherengitter hinausragende Teil der Bohrnadel wird in das Holz getrieben. Das Scherengitter liegt während des gesamten Vorgangs am Holz an.

Beim Zusammenklappen des Scherengitters wird der Winkel der Streben zur Bohrnadel größer und damit verbreitert sich das Scherengitter. Alle Durchtrittsöffnungen bewegen sich gleichmäßig aufeinander zu, so dass das Verhältnis der Abstände der Durchtrittsöffnungen und damit der Führungsstellen immer gleich bleibt.

Vorteilhaft liegt bei mehreren oder allen der Stützstrukturen zumindest einenends ein Formkörper vor, wobei in eingeklapptem Zustand des Scherengitters die Formkörper, die auf derselben Seite der Ebene E vorliegen, bündig aneinander anliegen, damit das Scherengitter sich in eingeklapptem Zustand nicht verkantet. Die Formkörper sind bevorzugt Nutensteine. Sie sind so konzipiert, dass sie sich nicht verdrehen können, linear führen und in zusammengefahrenem Zustand äußerst kompakt sind. Geeigneter Weise werden die Nutensteine, die paarig angeordnet sind, oben und unten um jeweils 180° zueinander verdreht montiert werden.

Eine Stützstruktur kann mit dem Abschnitt, der die Durchtrittsöffnung für die Bohrnadel aufweist, unmittelbar auf einer der Streben, die ein Kreuz des Scherengitters bilden, befestigt sein, so dass der Abschnitt der Stützstruktur, der das Gelenk aufweist, innerhalb der Streben zu liegen kommt bzw., dass die Streben den Gelenkabschnitt umfassen.

Die Stützstruktur mit Gelenk kann vorteilhaft zwei- oder mehrteilig sein, so dass alle Teile - ebenso wie die des Scherengitters - einfach gefertigt werden und verbaut werden können.

So kann bspw. ein erster gewindebildender Abschnitt der Stützstruktur eine Hülse sein, mit der eine der beiden sich kreuzenden Streben verbunden ist, und ein zweiter gewindebildender Abschnitt ist ein Stift, mit dem die zweite Strebe verbunden ist, wobei der Stift drehbar in Eingriff mit der Hülse steht.

Konkret: Wenn etwa ein erster Abschnitt der Stützstruktur als Stift ausgebildet ist, um einen unteren Abschnitt des Gewindes zu bilden, kann der Stift im Montagezustand dann durch die untere und die darüber liegende Strebe gesteckt werden um durch Verschrauben in einer Hülse aufgenommen zu werden, die den nächsten (zweiten) gewindebildenden Abschnitt bildet, der sich über die obere Strebe hinaus erstreckt und der sich einstückig oder durch Verbinden in den Abschnitt mit der Durchtrittsöffnung erstreckt.

Wird diese Idee weitergebildet, so kann der Abschnitt mit der Durchtrittsöffnung anderenends (abgewandt von der Gewindeseite) wieder in einen Gewindestift münden, der von einem Formkörper aufgenommen wird, der ein Innengewinde hat, um damit vom Abschnitt mit der Durchtrittsöffnung aufgenommen zu werden.

In noch einer weiteren Ausführungsform kann auch der erste Abschnitt der Stützstruktur, der als Stift ausgebildet ist, um einen unteren Abschnitt des Gewindes zu bilden, an seiner gewindeabgewandten Seite noch ein Gewinde haben oder als Hülse ausgebildet sein, von einem Formkörper aufgenommen werden, der ein Innengewinde hat, oder einen Formkörper entsprechend aufzunehmen.

Die Gestaltung der Bauteile mit korrespondierenden Gewinden ist grundsätzlich auch umkehrbar.

Weiter können die Bauteile auch auf andere Weise miteinander verbunden werden; etwa durch Verpressen, Verrasten oder andere, dem Fachmann bekannte Weise.

Zu "untere" und "obere": "Obere Seite" ist die Seite des Gelenks oder der Stützstruktur die das bei bodenparalleler Orientierung der Ebene E des Scherengitters nach oben zeigt; "untere Seite" zeigt in dem Fall nach unten. Dieses Verständnis von oben und unten gilt generell und auch, wenn die Vorrichtung in beliebiger Neigung eingesetzt wird.

Das untere Ende der Stützstruktur kann auch zugleich mit dem Gelenkabschnitt enden, so dass kein freies Ende aus dem Scherengitter herausragt.

In noch einer Ausführungsform ist möglich, dass die Stützstruktur direkt mit dem Scherengitter verbunden wird, in dem es an dieses angeschraubt oder mit ihm vernietet wird.

In einer Nadelführungsvorrichtung kann die Scherengittervorrichtung mit einem Vorschubantrieb eines vorbestimmten Bohrwiderstandsmessgeräts in Wirkverbindung stehen, so dass das Scherengitter durch den Vorschubantrieb von dem eingeklappten Zustand in den ausgeklappten Zustand überführbar ist. Dazu kann der Vorschubantrieb mit den Enden der am Rand liegenden Streben, die zum Bohrwiderstandmessgerät gerichtet sind, verbunden sein. So können die Enden dieser Streben auseinander gedrückt und damit kann über die Gelenkverbindungen das Scherengitter eingeklappt werden oder die Enden werden zusammen gedrückt und damit das Scherengitter ausgeklappt.

In noch einer weiteren Ausführungsform weist die Scherengittervorrichtung zwei Scherengitter auf, deren Ebenen E in aufgeklapptem Zustand parallel zueinander angeordnet sind. Dabei ist jeweils ein Gelenk des einen Scherengitters über eine gemeinsame Stützstruktur mit dem korrespondierenden Gelenk des anderen Scherengitters verbunden. Die Scherengitter sind bevorzugt derart angeordnet, dass die Durchtrittsöffnungen der Stützstruktur der Gelenke zwischen den beiden Scherengittern liegen. Die beiden Scherengitter einer solchen Anordnung sollten die gleiche Form aufweisen, so dass sich die Gelenkpunkte in einer Projektion auf eine der Ebenen E decken und damit auch die Streben die gleiche Länge aufweisen und sich beim Einklappen gleich schnell bewegen. Die Stützstruktur der Gelenke ist dann normal zu den beiden Ebenen E ausgerichtet.

Durch die Anordnung von zwei Scherengittern kann eine höhere Biegesteifigkeit erreicht werden, da das Flächenträgheitsmoment vergrößert wird, da die Scherengitter beidseitig von der Bohrnadel angeordnet sind und weiter von der Bohrnadel beabstandet sein können.

In einer alternativen Ausführungsform der Nadelführungsvorrichtung ist die Abstützvorrichtung eine Antriebsvorrichtung, die zwei parallel nebeneinander angeordnete, zusammenwirkende Kraftübertragungselemente aufweist.

Die Antriebsvorrichtung kann durch einen Kettenantrieb oder durch einen Riemenantrieb gebildet werden. Dabei weist der Kettenantrieb als Kraftübertragungselemente zwei Ketten, der Riemenantrieb zwei Zahnriemen auf.

In einer Aufführungsform sind die zwei Ketten oder die zwei Zahnriemen einenends um ein Ritzel und anderenends um ein Antriebsritzel gespannt. Die Antriebsritzel sind dazu ausgebildet, durch einen Vorschubantrieb des vorbestimmten Bohrwiderstandsmessgeräts angetrieben zu werden. Die Nadelhalterung ist an der Antriebsvorrichtung befestigt und wird durch die Antriebsvorrichtung von einer hinteren Position in eine vordere Position überführt. In der hinteren Position ist die Nadelhalterung an dem Ende der Antriebsvorrichtung positioniert, die in einem Benutzungszustand bohrwiderstandsmessgeräteseitig liegt. In der vorderen Position ist die Nadelhalterung am anderen Ende der Antriebsvorrichtung positioniert.

Die Kraftübertragungselemente weisen Führungselemente auf, und jeweils ein Führungselement des einen Kraftübertragungselements steht mit einem Führungselement des anderen Kraftübertragungselements in Wirkverbindung. Beide Führungselemente zusammen bilden eine Einfassung für die vorbestimmte Bohrnadel.

Die Bohrnadel liegt zwischen den beiden Kraftübertragungselementen, die auf gegenüberliegenden Seiten der Bohrnadel angeordnet sind. Die Bohrnadel wird mit der Nadelhalterung, in die sie eingespannt ist, von der hinteren Position in die vordere Position verschoben. Dabei wird die Bohrnadel, die in der hinteren Position vollständig zwischen den Kraftübertragungselementen liegt und von allen Führungselementen abgestützt wird, die zwischen den Kraftübertragungselementen angeordnet sind, nach und nach in die vordere Position bewegt, in der sie weit über die Antriebsvorrichtung hinaus ragt. Während der Bewegung lösen sich nacheinander die in Wirkverbindung stehenden Führungselemente voneinander, wenn sie an der Stelle des Ritzels voneinander weg bewegt werden. Die Bohrnadel wird so von immer weniger Führungselementen eingefasst und geführt. Dabei bewegen sich die Kraftübertragungselemente mit der Bohrnadel mit, so dass es keine Relativbewegung zwischen der Bohrnadel und den Kraftübertragungselementen gibt. Damit entsteht auch keine Reibung in Längsrichtung der Bohrnadel. In der Anwendung wird durch diese Bewegung die Bohrnadel in das Holz getrieben.

Die Bewegung wird durch ein synchrones Antreiben der Antriebsritzel erreicht, wobei die Drehrichtungen entgegengesetzt sind. Die Kraftübertragungselemente werden durch die Antriebsritzel angetrieben und bewegen die Nadelhalterung.

In einer noch weiteren Ausführungsform sind die zwei Ketten aus Kettengliedern gebildet, die Bolzen und Laschen aufweisen. Die Aufnahme der Bohrnadel wird durch die Bolzen der Kettenglieder gebildet. Dabei können die Bolzen eine Innenwölbung aufweisen, die einer Negativform der Bohrnadel entspricht. Die Bohrnadel wird dann von den Bolzen jeweils in die Richtung der Kette abgestützt.

Die beiden Ketten können derart nebeneinander verlaufen, dass sich die Laschen der Kettenglieder verzahnen. Das bedeutet, dass die Kettenglieder der beiden Ketten versetzt zueinander angeordnet sind, so dass eine konkave Stelle des Kettenglieds bzw. der Lasche eine konvexe Stelle des Kettenglieds der anderen Kette aufnimmt. Eine Relativbewegung der beiden Ketten ist dann nicht mehr möglich. Beide Ketten müssen mit der gleichen Geschwindigkeit bewegt werden.

Nach einer anderen möglichen Ausführungsform weisen die Zahnriemen auf ihrer nicht verzahnten Seite Haken auf. Die Haken des einen Zahnriemens greifen in die Haken des anderen Zahnriemens ein. Durch das Eingreifen der Haken ineinander wird auch hier eine Relativbewegung der beiden Zahnriemen zueinander verhindert.

Die Haken weisen jeweils eine von dem Riemen weg gerichtete Aussparung auf. Diese Aussparungen überschneiden sich in der Eingriffsposition und bilden einen runden Durchgang als Einfassung für die vorbestimmte Bohrnadel. Die Bohrnadel wird also auf der einen Seite von der Aussparung des Hakens des einen Riemens und auf der anderen Seite von der Aussparung des Hakens des anderen Riemens geführt.

Ein erfindungsgemäßes Bohrwiderstandsmessgerät zur Beschaffenheitsuntersuchung von Holz weist eine Nadelführungsvorrichtung in einer der aufgeführten Ausführungsformen auf.

Das Bohrwiderstandsmessgerät kann weiter einen Vorschubantrieb aufweisen. Dieser Vorschubantrieb kann mit dem Antriebsritzel einer Antriebsvorrichtung kraftübertragend gekoppelt werden und/oder so mit dem Scherengitter kraftübertragend verbunden werden, dass das Scherengitter durch den Vorschubantrieb von dem eingeklappten Zustand in den ausgeklappten Zustand überführt wird.

Ein erfindungsgemäßes Verfahren zur Beschaffenheitsuntersuchung von Holz erfolgt unter Verwendung einer erfindungsgemäßen Nadelführungsvorrichtung mit einer Scherengittervorrichtung und umfasst folgende Schritte:
- Bereitstellen eines Bohrwiderstandsmessgeräts mit der Nadelführungsvorrichtung und eingespannter Bohrnadel,
- Überführen des Scherengitters aus Streben der Scherengittervorrichtung der Abstützvorrichtung der Bohrnadel der Nadelführungsvorrichtung in einem ausgeklappten Zustand, dann
- Ansetzen der Nadelführungsvorrichtung mit der Bohrnadel an ein zu untersuchendes Objekt aus Holz, dann
- Einführen der Bohrnadel in das Objekt aus Holz und dabei zugleich Überführen des Scherengitters in den eingeklappten Zustand.

Ein weiteres erfindungsgemäßes Verfahren zur Beschaffenheitsuntersuchung von Holz erfolgt unter Verwendung einer erfindungsgemäßen Nadelführungsvorrichtung mit einer Antriebsvorrichtung und umfasst folgende Schritte:
- Bereitstellen eines Bohrwiderstandsmessgeräts mit der Nadelführungsvorrichtung und eingespannter Bohrnadel und Führen der Bohrnadel in der Abstützvorrichtung, als Antriebsvorrichtung einen Kettenantrieb, der als Kraftübertragungselemente zwei Ketten aufweist, oder in der Abstützvorrichtung, die als Antriebsvorrichtung einen Riemenantrieb, der als Kraftübertragungselemente zwei Zahnriemen aufweist,
- Positionieren der Nadelhalterung in der hinteren Position an der Antriebsvorrichtung,
- Ansetzen der Nadelführungsvorrichtung mit der Bohrnadel an ein zu untersuchendes Objekt aus Holz, dann Einführen der Bohrnadel in das Objekt aus Holz, dabei zugleich
- Überführen der Nadelhalterung in die vordere Position.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig. 1**: eine Draufsicht auf eine Nadelführungsvorrichtung mit einer Scherengittervorrichtung mit eingespannter Bohrnadel,
- **Fig. 2**: eine perspektivische Ansicht der Nadelführungsvorrichtung aus Fig. 1,
- **Fig. 3**: eine perspektivische Vorderansicht des vorderen Teils der Scherengittervorrichtung aus Fig. 1,
- **Fig. 4**: eine Draufsicht auf eine paarige Nutenstein-Anordnung
- **Fig. 5**: eine Draufsicht auf eine Nadelführungsvorrichtung mit einem Riemenantrieb mit eingespannter Bohrnadel,
- **Fig. 6**: eine perspektivische Vorderansicht des vorderen Teils des Riemenantriebs aus Fig. 4,
- **Fig. 7**: eine Draufsicht auf eine Nadelführungsvorrichtung mit einem Kettenantrieb mit eingespannter Bohrnadel,
- **Fig. 8**: eine perspektivische Vorderansicht des vorderen Teils des Kettenantriebs aus Fig. 6.

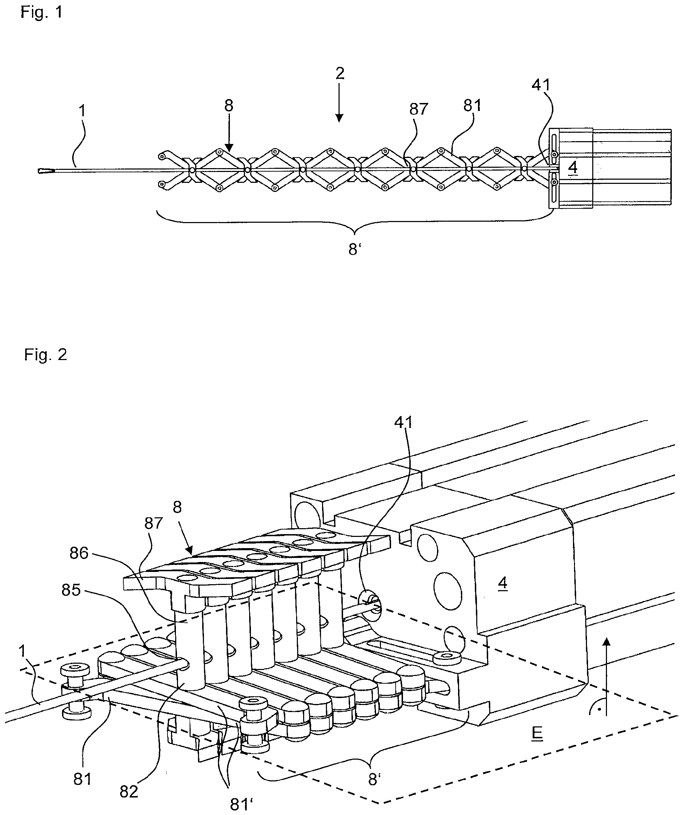

Die erfindungsgemäße Vorrichtung bezieht sich auf eine Nadelführungsvorrichtung 2, die in mehreren Ausführungen in den **Figuren 1****,** **5** **und** **7** dargestellt ist.

Die **Figuren 1 bis 3** zeigen eine Nadelführungsvorrichtung 2 mit einer Scherengittervorrichtung 8, in der die Bohrnadel 1 eingespannt ist. Die Scherengittervorrichtung 8 hat ein Scherengitter 8', das sich in Längsrichtung der Bohrnadel 1 erstreckt. Das Scherengitter 8' ist aus Streben 81 zusammengesetzt, wobei jeweils zwei sich kreuzende Streben 81 in ihrer Mitte durch ein Gelenk 82 verbunden sind, das einen Abschnitt der Stützstruktur 86 bildet, die sich normal zu der durch das Scherengitter 8' aufgespannten Ebene E, siehe **Fig. 2**, erstreckt. In die Stützstruktur 86 ist eine Durchtrittsöffnung 85 eingebracht, in der die Bohrnadel 1 aufgenommen wird. Dazu sind die Durchtrittsöffnungen 85 der Stützstrukturen 86 alle so ausgerichtet, dass sie miteinander und mit der Öffnung 41 der Nadelhalterung 4 fluchten. **Fig. 2** zeigt auch die Öffnung 41 der Nadelhalterung 4, in die die Bohrnadel 1 eingeführt ist. In der Nadelhalterung 4 wird die Bohrnadel 1 durch ein Bohrfutter gehalten.

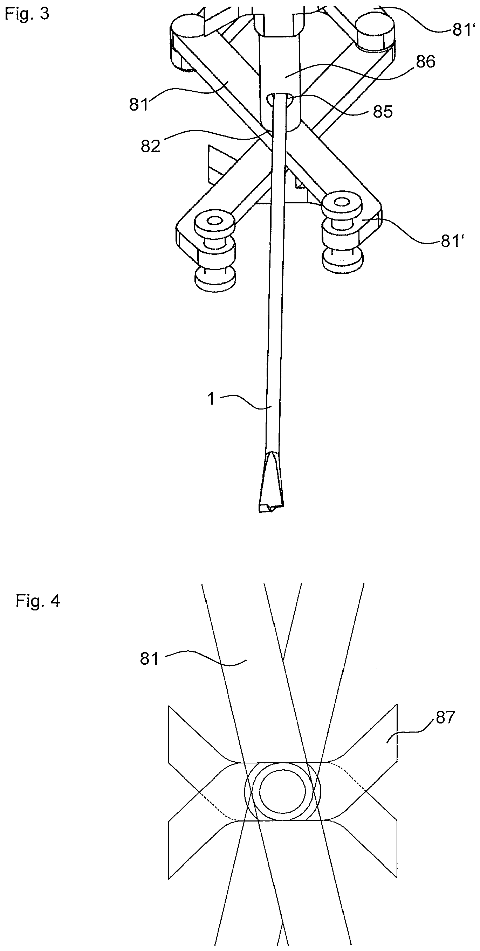

**Fig. 2** zeigt auch, dass die Enden 81' der Streben 81 des Scherengitters 8' mit den Enden 81' der nächstliegenden Streben 81 gelenkig verbunden sind. Die Enden 81' der Streben 81, die das Ende des Scherengitters 8' an der Nadelhalterung 4 bilden, sind an der Nadelhalterung 4 befestigt. Dazu sind die Enden 81' eines Strebenpaares aus zwei zusammengehörigen Streben 81 mit Stiften verbunden, die in Langlöchern geführt sind, so dass die Enden 81' der Streben 81 sich nur eindimensional aufeinander zu und voneinander weg bewegen können. Damit kann das Scherengitter 8' eingeklappt und ausgeklappt werden. **Fig. 2** zeigt das Scherengitter 8' in eingeklappten Zustand. Die Formkörper 87, die an den freien oberen Enden der Stützstrukturen 86 und auch unterhalb der Ebene E an den unteren Abschnitten der Stützstrukturen 86 vorliegen, Siehe auch **Fig. 4****,** die eine paarige Anordnung zweier Nutensteine zeigt, liegen bündig aneinander an. In **Fig. 1****,** in der das Scherengitter 8' ausgeklappt ist, sind die Formkörper 87 voneinander beabstandet. Die Formkörper 87, die bevorzugt Nutensteine 87 sind, sind so konzipiert, dass sie sich nicht verdrehen können, linear führen und in zusammengefahrenem Zustand äußerst kompakt sind. Hierzu ist es wichtig, dass die Nutensteine 87, die paarig angeordnet sind und oben und unten jeweils 180° zueinander verdreht montiert werden, wie **Fig. 4** zeigt.

**Fig. 3** zeigt nur einen Ausschnitt der Scherengittervorrichtung 8 mit den vordersten beiden Streben 81 und einem mittigen Gelenk 82 mit Stützstruktur 86, in der durch die Durchtrittsöffnung 85 die Bohrnadel 1 verläuft. **Fig. 3** zeigt das Scherengitter 8' in beinahe vollständig ausgeklapptem Zustand. Die Bohrnadel 1 steht nur ein kleines Stück über das Scherengitter 8' hinaus. Beim Ausklappen und Einklappen des Scherengitters 8' bleibt die Anzahl der Durchtrittsöffnungen 85, in denen die Bohrnadel 1 geführt wird, gleich. Die Abstände werden gleichmäßig größer oder kleiner. In der dargestellten Variante sind die Abstände zwischen den Durchtrittsöffnungen 85 immer gleich groß. Es sind jedoch auch Varianten möglich, in denen die Abstände unterschiedlich sind. Doch auch dann bleibt das Verhältnis der Abstände während der Klappbewegung immer gleich groß.

Nicht in den Figuren gezeigt sind weitere Ausführungsformen, in denen die Stützstrukturen 86 mit Gelenken 82 mehrteilig ausgebildet sind und sich durch eine Gewindeverbindung zusammenschrauben lassen.

Ebenfalls nicht figurativ gezeigt ist eine Ausführungsform, in der die Scherengittervorrichtung 8 zwei Scherengitter 8' aufweist und die Bohrnadel 1 zwischen beiden Scherengittern 8' geführt wird.

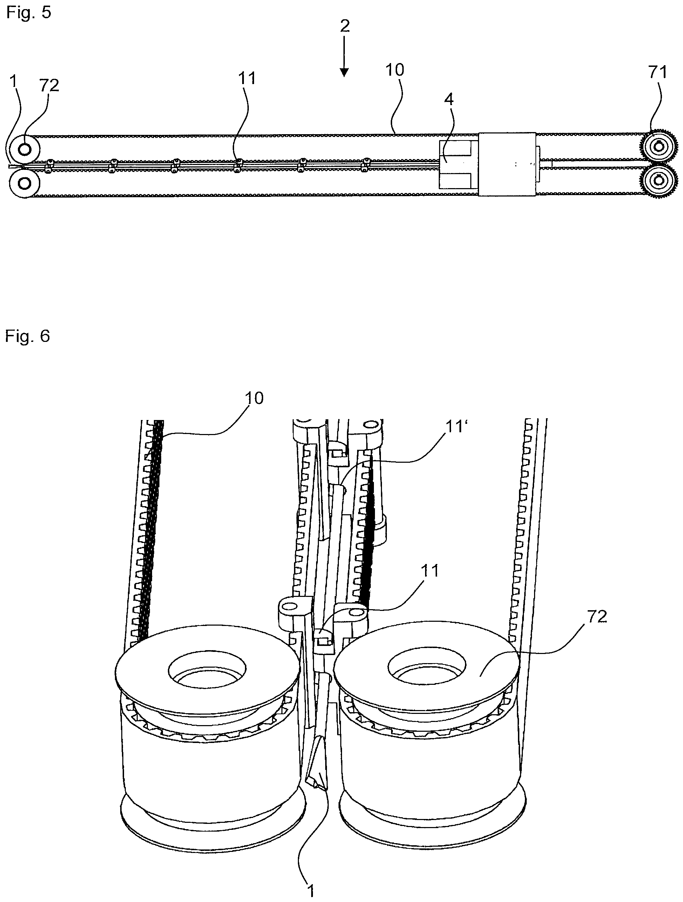

Die **Figuren 5** und **6** zeigen eine Nadelführungsvorrichtung 2 mit einem Riemenantrieb, bei dem die Nadelhalterung 4 durch die Zahnriemen 10 bewegt wird. Die Zahnriemen 10 sind um ein Ritzel 72 und ein Antriebsritzel 71 gespannt und werden über das Antriebsritzel 71 angetrieben. **Fig. 5** zeigt den Verlauf der Zahnriemen, die an der einander zugewandten Seite parallel zueinander verlaufen. Die Bohrnadel 1 verläuft zwischen den beiden Zahnriemen 10. Für die Führung der Bohrnadel 1 sind an der unverzahnten Seite der Zahnriemen 10 Haken 11 angebracht, die jeweils eine zur Bohrnadel 1 hin offene Aussparung 11' aufweisen. Am geschlossenen Ende ist diese Aussparung 11' halbrund ausgeformt, so dass die Bohrnadel 1 in dieser Aussparung 11' zur einen Seite hin abgestützt wird. Zwischen den beiden Zahnriemen 11 überschneiden sich die beiden Aussparungen 11' der Haken 11 der beiden Zahnriemen 10, so dass durch die beiden Aussparungen 11' hindurch ein runder Durchgang für die Bohrnadel 1 verbleibt. Durch die Durchgänge aller Hakenpaare verläuft die Bohrnadel. Dies zeigt **Fig. 6**, in der ein Ausschnitt des Riemenantriebs dargestellt ist, in dem die vorderen beiden Hakenpaare zu sehen sind.

**Fig. 6** zeigt auch, dass jeweils zwei Haken 11 ineinander greifen. Dies dient dem Zweck, dass diese Verbindung ein Verrutschen der beiden Zahnriemen 11 zueinander und damit eine Relativbewegung zueinander verhindert.

In der Vorschubbewegung der Bohrnadel 1 werden die Zahnriemen 11 und die Bohrnadel 1 gemeinsam bewegt. Es kommt damit zu keiner Relativbewegung in Längsrichtung der Bohrnadel 1 zwischen der Bohrnadel 1 und der Nadelführungsvorrichtung 2. Damit entsteht auch keine Reibung. Reibung tritt nur an den Aussparungen 11' auf, in denen die Bohrnadel geführt wird, da sich die Bohrnadel 1 dreht.

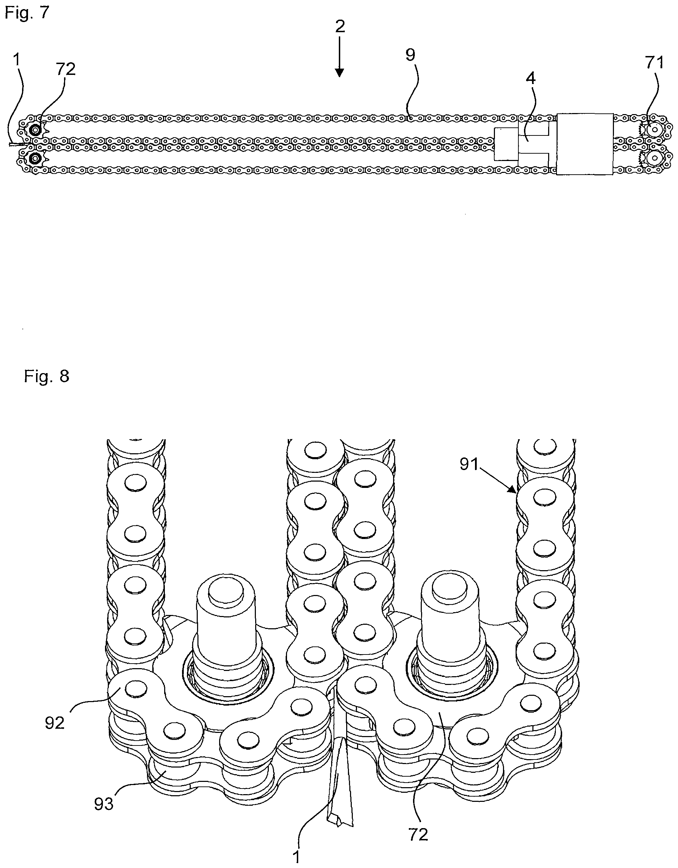

Die **Figuren 7** und **8** zeigen eine Nadelführungsvorrichtung 2 mit einem Kettenantrieb, bei dem die Nadelhalterung 4 durch die Ketten 9 bewegt wird. Die Kette ist aus einzelnen Kettengliedern 91 aufgebaut, die aus Bolzen 93 und Laschen 92 bestehen. Dabei sind die jeweils zwei Laschen 92 eines Kettenglieds 91 mit den beiden Laschen des nächsten Kettenglieds 91 durch einen Bolzen 93 gelenkig verbunden. Dadurch kann die Kette 9 sich an runde Formen anpassen.

**Fig. 7** zeigt die Anordnung der Kette 9, die, wie auch der Zahnriemen 10, um ein Ritzel 72 und ein Antriebsritzel 71 verläuft. Antriebsritzel 71 und Ritzel 72 sind dazu an die Kette 9 angepasst, so dass die Zähne des Ritzels 72 bzw. des Antriebsritzels 71 in die Kettenglieder eingreifen können.

**Fig. 8** zeigt, dass die Bohrnadel 1 zwischen den nebeneinander verlaufenden Kettenabschnitten verläuft und sich an den Bolzen 93 der Kettenglieder 91 abstützt. Nicht in den Figuren gezeigt ist, dass die Bolzen 93 zur Führung der Bohrnadel 1 speziell geformt sein können.

In den **Figuren 7** und **8** ist auch gezeigt, wie die Ketten 9 nebeneinander verlaufen, damit die Kettenglieder 91 ineinander eingreifen. Dazu sind die Ketten 9 um ein halbes Kettenglied 91 versetzt zueinander angeordnet. So kommt die konvexe Rundung der Lasche 92 um den Bolzen 93 in einem konkaven Abschnitt der Lasche 92 zwischen den Bolzen 93 zu liegen. Diese Position der Kettenglieder 91 zueinander verhindert eine Relativbewegung der beiden Ketten 9 zueinander und es wird eine synchrone Bewegung der Ketten 9 gesichert.

## Patentansprüche

1. Nadelführungsvorrichtung (2) für ein vorbestimmtes Bohrwiderstandsmessgerät, die dazu ausgebildet ist, eine vorbestimmte Bohrnadel (1) zu führen und mit einem Antrieb des Bohrwiderstandsmessgeräts operativ gekoppelt zu werden,
wobei die Nadelführungsvorrichtung (2) eine Abstützvorrichtung und eine Nadelhalterung (4) aufweist, und wobei
- die Nadelhalterung (4) ein Bohrfutter aufweist, das dazu ausgebildet ist, die vorbestimmte Bohrnadel (1) aufzunehmen, und
- die Abstützvorrichtung sich in Längsrichtung der vorbestimmten Bohrnadel (1) erstreckt,
**dadurch gekennzeichnet, dass**
die Abstützvorrichtung zumindest eine Scherengittervorrichtung (8) mit einem Scherengitter (8') aus Streben (81) aufweist.

2. Nadelführungsvorrichtung (2) nach Anspruch 1,
**dadurch gekennzeichnet, dass**
das Scherengitter (8') in einen eingeklappten und in einen ausgeklappten Zustand überführbar ist und eine Ebene E aufspannt,
wobei jeweils zwei sich kreuzende Streben (81) mittels einer Stützstruktur (86) mit Gelenk (82) gelenkig miteinander verbunden sind,
und die Stützstrukturen (86) sich normal zu der durch das Scherengitter aufgespannten Ebene E weg erstrecken und
wobei jede Stützstruktur (86) eine Durchtrittsöffnung (85) als eine Aufnahme für die vorbestimmte Bohrnadel (1) aufweist und die Durchtrittsöffnungen (85) aller Stützstrukturen (86) miteinander und mit einer Öffnung (41) der Nadelhalterung (4) fluchten.

3. Nadelführungsvorrichtung (2) nach Anspruch 2,
**dadurch gekennzeichnet, dass**
das Scherengitter (8') in dem ausgeklapptem Zustand eine Länge aufweist, die so ausgewählt ist, dass sich die vorbestimmte Bohrnadel (1) in einer in dem Bohrfutter aufgenommenen Position nicht über das Scherengitter (8') hinaus erstreckt, und das Scherengitter (8') in dem eingeklappten Zustand eine Länge aufweist, die so ausgewählt ist, dass die vorbestimmte Bohrnadel (1) mindestens zur Hälfte ihrer Länge über das Scherengitter (8') hinaus ragt.

4. Nadelführungsvorrichtung (2) nach Anspruch 2 oder 3,
**dadurch gekennzeichnet, dass**
zumindest bei einer Mehrzahl der Stützstrukturen (86) zumindest einenends ein Formkörper (87) vorliegt,
wobei in eingeklapptem Zustand des Scherengitters (8') die Formkörper (87), die auf derselben Seite der Ebene E vorliegen, bündig aneinander anliegen.

5. Nadelführungsvorrichtung (2) nach zumindest einem der Ansprüche 2 bis 4,
**dadurch gekennzeichnet, dass**
die Stützstruktur (86) mit ihrem die Durchtrittsöffnung (85) aufweisenden Abschnitt unmittelbar auf einer der Streben (81) befestigt ist und der das Gelenk (82) aufweisende Abschnitt innerhalb der Streben zu liegen kommt.

6. Nadelführungsvorrichtung (2) nach zumindest einem der Ansprüche 2 bis 5,
**dadurch gekennzeichnet, dass**
die Stützstruktur (86) mit Gelenk (82) zwei- oder mehrteilig ist und die Abschnitte durch Verschrauben miteinander verbindbar sind,
wobei bevorzugt ein erster gewindebildender Abschnitt der Stützstruktur (86) eine Hülse ist, mit der eine der beiden sich kreuzenden Streben (81) verbunden ist und ein zweiter gewindebildender Abschnitt ein Stift ist, mit dem die zweite Strebe (81) verbunden ist, wobei der Stift drehbar in Eingriff mit der Hülse steht.

7. Nadelführungsvorrichtung (2) nach zumindest einem der Ansprüche 1 bis 6,
**dadurch gekennzeichnet, dass**
die Scherengittervorrichtung (8)
- mit einem Vorschubantrieb des vorbestimmten Bohrwiderstandsmessgeräts in Wirkverbindung steht, wobei das Scherengitter (8') durch den Vorschubantrieb von dem eingeklappten Zustand in den ausgeklappten Zustand überführbar ist und/oder zwei Scherengitter (8') aufweist, deren Ebenen E in aufgeklapptem Zustand parallel zueinander angeordnet sind, wobei jeweils ein Gelenk (82) des einen Scherengitters (8') über eine gemeinsame Stützstruktur (86) mit dem korrespondierenden Gelenk (82) des anderen Scherengitters (8') verbunden ist,
wobei bevorzugt die Scherengitter (8') so angeordnet sind, dass die Durchtrittsöffnungen (85) der Stützstrukturen (86) zwischen den beiden Scherengittern (8') liegen.

8. Nadelführungsvorrichtung (2) für ein vorbestimmtes Bohrwiderstandsmessgerät, die dazu ausgebildet ist, eine vorbestimmte Bohrnadel (1) zu führen und mit einem Antrieb des Bohrwiderstandsmessgeräts operativ gekoppelt zu werden,
wobei die Nadelführungsvorrichtung (2) eine Abstützvorrichtung und eine Nadelhalterung (4) aufweist, und wobei
- die Nadelhalterung (4) ein Bohrfutter aufweist, das dazu ausgebildet ist, die vorbestimmte Bohrnadel (1) aufzunehmen, und
- die Abstützvorrichtung sich in Längsrichtung der vorbestimmten Bohrnadel (1) erstreckt,
**dadurch gekennzeichnet, dass**
die Abstützvorrichtung eine Antriebsvorrichtung ist, die zwei parallel nebeneinander angeordnete, zusammenwirkende Kraftübertragungselemente aufweist.

9. Nadelführungsvorrichtung (2) nach Anspruch 8,
**dadurch gekennzeichnet, dass**
- die Antriebsvorrichtung durch einen Kettenantrieb, der als Kraftübertragungselemente zwei Ketten (9) aufweist, oder
- die Antriebsvorrichtung durch einen Riemenantrieb, der als Kraftübertragungselemente zwei Zahnriemen (10) aufweist,
gebildet werden.

10. Nadelführungsvorrichtung (2) nach Anspruch 9,
**dadurch gekennzeichnet, dass**
die zwei Ketten (9) oder zwei Zahnriemen (10) jeweils einenends um ein Ritzel (72) und anderenends um ein Antriebsritzel (71) gespannt sind,
wobei die Antriebsritzel (71) dazu ausgebildet sind, durch einen Vorschubantrieb des vorbestimmten Bohrwiderstandsmessgeräts angetrieben zu werden, und
- wobei die Nadelhalterung (4) an der Antriebsvorrichtung befestigt ist und durch die Antriebsvorrichtung von einer hinteren Position, in der die Nadelhalterung (4) am dem Ende der Antriebsvorrichtung positioniert ist, die in einem Benutzungszustand bohrwiderstandsgeräteseitig liegt, in eine vordere Position, in der die Nadelhalterung am anderen Ende der Antriebsvorrichtung positioniert ist, überführbar ist, und
- wobei die Kraftübertragungselemente Führungselemente aufweisen und jeweils ein Führungselement des einen Kraftübertragungselements mit einem Führungselement des anderen Kraftübertragungselements in Wirkverbindung steht und eine Einfassung für die vorbestimmte Bohrnadel (1) bildet.

11. Nadelführungsvorrichtung (2) nach Anspruch 8 oder 9,
**dadurch gekennzeichnet, dass**
die zwei Ketten (9) Kettenglieder (91) aufweisen, deren Kettenglieder (91) Bolzen (93) und Laschen (92) aufweisen, wobei die Einfassung für die vorbestimmte Bohrnadel (1) durch die Bolzen (93) der Kettenglieder (91) gebildet ist, wobei die Bolzen (93) eine Innenwölbung aufweisen, die einer Negativform der Bohrnadel (1) entspricht,
und wobei bevorzugt
die beiden Ketten (9) derart nebeneinander verlaufen, dass sich die Laschen (92) der Kettenglieder (91) verzahnen.

12. Nadelführungsvorrichtung (2) nach Anspruch 9,
**dadurch gekennzeichnet, dass**
die Zahnriemen (10) des Riemenantriebs auf ihrer nicht verzahnten Seite Haken (11) aufweisen, wobei die Haken (11) des einen Zahnriemens (10) in die Haken des anderen Zahnriemens (10) eingreifen und die Haken (11) eine von dem Riemen weg gerichtete Aussparung (11') aufweisen, die sich in einer Eingriffsposition überschneiden und einen runden Durchgang als Einfassung für die vorbestimmte Bohrnadel (1) bilden.

13. Bohrwiderstandsmessgerät zur Beschaffenheitsuntersuchung von Holz,
**dadurch gekennzeichnet, dass**
das Bohrwiderstandsmessgerät eine Nadelführungsvorrichtung (2) nach einem der Ansprüche 1 bis 12 aufweist.

14. Bohrwiderstandsmessgerät nach Anspruch 13,
**dadurch gekennzeichnet, dass**
das Bohrwiderstandsmessgerät einen Vorschubantrieb aufweist, der dazu ausgebildet ist,
- an die Antriebsritzel (71) kraftübertragend gekoppelt zu werden und/oder
- das Scherengitter (8') von dem eingeklappten Zustand in den ausgeklappten Zustand zu überführen.

15. Verfahren zur Beschaffenheitsuntersuchung von Holz unter Verwendung einer Nadelführungsvorrichtung nach zumindest einem der Ansprüche 1 bis 7,
**umfassend die Schritte**
- Bereitstellen eines Bohrwiderstandsmessgeräts mit der Nadelführungsvorrichtung (2) und eingespannter Bohrnadel (1)
- Überführen des Scherengitters (8') aus Streben (81) der Scherengittervorrichtung (8) der Abstützvorrichtung der Bohrnadel (1) der Nadelführungsvorrichtung (2) in einem ausgeklappten Zustand, dann
- Ansetzen der Nadelführungsvorrichtung (2) mit der Bohrnadel (1) an ein zu untersuchendes Objekt aus Holz, dann
- Einführen der Bohrnadel (1) in das Objekt aus Holz und dabei zugleich Überführen des Scherengitters (8') in den eingeklappten Zustand.

16. Verfahren zur Beschaffenheitsuntersuchung von Holz unter Verwendung einer Nadelführungsvorrichtung nach zumindest einem der Ansprüche 8 bis 12,
**umfassend die Schritte**
- Bereitstellen eines Bohrwiderstandsmessgeräts mit der Nadelführungsvorrichtung (2) und eingespannter Bohrnadel (1) und Führen der Bohrnadel (1) in der Abstützvorrichtung, als Antriebsvorrichtung einen Kettenantrieb, der als Kraftübertragungselemente zwei Ketten (9) aufweist, oder in der Abstützvorrichtung, die als Antriebsvorrichtung einen Riemenantrieb, der als Kraftübertragungselemente zwei Zahnriemen (10) aufweist,
- Positionieren der Nadelhalterung (4) in der hinteren Position an der Antriebsvorrichtung,
- Ansetzen der Nadelführungsvorrichtung (2) mit der Bohrnadel (1) an ein zu untersuchendes Objekt aus Holz, dann Einführen der Bohrnadel (1) in das Objekt aus Holz, dabei zugleich
- Überführen der Nadelhalterung (4) in die vordere Position.

## Claims

1. Needle-guiding device (2) for a specified drilling resistance measurement unit that is configured to guide a specified drilling needle (1) and to be operatively coupled with a drive of the drilling resistance measurement unit,
wherein the needle-guiding device (2) comprises a support device and a needle holder (4), and wherein
- the needle holder (4) comprises a drill chuck that is configured to receive the specified drilling needle (1), and
- the support device extends in longitudinal direction of the specified drilling needle (1),
**characterized in that**
the support device comprises at least one scissor-type extension arm device (8) with a scissor-type extension arm (8') of stays (81).

2. Needle-guiding device (2) according to claim 1,
**characterized in that**
the scissor-type extension arm (8') is transferable into a folded and into an extended state and defines a plane E,
wherein two stays (81) crossing each other are respectively connected to each other pivotably by means of a support structure (86) with joint (82),
and the support structures (86) extend away normal relative to the plane E defined by the scissor-type extension arm (8'), and
wherein each support structure (86) comprises a passage (85) as a receptacle for the specified drilling needle (1) and the passages (85) of all support structures (86) are aligned with each other and with an opening (41) of the needle holder (4).

3. Needle-guiding device (2) according to claim 2,
**characterized in that**
the scissor-type extension arm (8') in the extended state has a length that is selected such that the specified drilling needle (1), in a position received in the drill chuck, does not project past the scissor-type extension arm (8'),
and the scissor-type extension arm (8') in the folded state has a length that is selected such that the specified drilling needle (1) at least with half of its length projects past the scissor-type extension arm (8').

4. Needle-guiding device (2) according to claim 2 or 3,
**characterized in that**
at least for a plurality of the support structures (86) a shaped body (87) is present at least at one end,
wherein in the folded state of the scissor-type extension arm (8') the shaped bodies (87) that are positioned on the same side of the plane E, are resting flush against each other.

5. Needle-guiding device (2) according to at least one of the claims 2 to 4,
**characterized in that**
the support structure (86) with its section comprising the passage (85) is fastened immediately on one of the stays (81) and the section comprising the joint (82) is positioned inside the stays.

6. Needle-guiding device (2) according to at least one of the claims 2 to 5,
**characterized in that**
the support structure (86) with joint (82) is of a two-part or multi-part configuration and the sections are connectable to each other by screwing,
wherein preferably the first thread-constituting section of the support structure (86) is a sleeve with which one of the two stays (81) crossing each other is connected and a second thread-constituting section is a pin with which the second stay (81) is connected, wherein the pin is rotatably engaging the sleeve.

7. Needle-guiding device (2) according to at least one of the claims 1 to 6,
**characterized in that**
the scissor-type extension arm device (8)
- is in operative connection with a feed drive of the specified drilling resistance measurement unit, wherein the scissor-type extension arm (8') is transferable from the folded state into the extended state by means of the feed drive and/or comprises two scissor-type extension arms (8'), whose planes E in the extended state are arranged parallel to each other, wherein one joint (82) each of one scissor-type extension arm (8') is connected by means of a common support structure (86) with the corresponding joint (82) of the other scissor-type extension arm (8'), wherein preferably the scissor-type extension arms (8') are arranged such that the passages (85) of the support structures (86) are positioned between the two scissor-type extension arms (8').

8. Needle-guiding device (2) for a specified drilling resistance measurement unit that is configured to guide a specified drilling needle (1) and to be operatively coupled to a drive of the drilling resistance measuring unit,
wherein the needle-guiding device (2) comprises a support device and a needle holder (4), and wherein
- the needle holder (4) comprises a drill chuck that is configured to receive the specified drilling needle (1), and
- the support device extends in longitudinal direction of the specified drilling needle (1),
**characterized in that**
the support device is a drive device that comprises two interacting force transmitting elements that are arranged parallel adjacent to each other.

9. Needle-guiding device (2) according to claim 8,
**characterized in that**
- the drive device is formed by a chain drive that comprises two chains (9) as force transmitting elements, or
- the drive device is formed by a belt drive that comprises two toothed belts (10) as force transmitting elements.

10. Needle-guiding device (2) according to claim 9,
**characterized in that**
the two chains (9) or the two toothed belts (10) each are tensioned about a pinion (72) with one end and about a drive pinion (71) with the other end,
wherein the drive pinions (71) are configured to be driven by a feed drive of the specified drilling resistance measurement unit, and
- wherein the needle holder (4) is attached to the drive device and is transferable by means of the drive device from a rear position, in which the needle holder (4) is positioned at the end of the drive device that is positioned at the drilling resistance measurement unit in a state of use, into a forward position in which the needle holder (4) is positioned at the other end of the drive device, and
- wherein the force transmitting elements comprise guide elements and one guide element each of one force transmitting element is in operative connection with a guide element of the other force transmitting element and forms an enclosure for the specified drilling needle (1).

11. Needle-guiding device (2) according to claim 8 or 9,
**characterized in that**
the two chains (9) comprise chain members (91) whose chain members (91) comprise bolts (93) and links (92), wherein the enclosure for the specified drilling needle (1) is formed by the bolts (93) of the chain members (91), wherein the bolts (93) comprise an inward curvature that corresponds to a negative shape of the drilling needle (1),
and wherein preferably
the two chains (9) extend adjacent to each other in such a way that the links (92) of the chain members (91) are meshing.

12. Needle-guiding device (2) according to claim 9,
**characterized in that**
the toothed belts (10) of the belt drive comprise hooks (11) at their side without teeth, wherein the hooks (11) of one toothed belt (10) engage the hooks of the other toothed belt (10) and the hooks (11) comprise a cutout (11') facing away from the belt, which in an engagement position overlap each other and provide a round passage as enclosure for the specified drilling needle (1).

13. Drilling resistance measurement unit for investigating the nature of wood,
**characterized in that**
the drilling resistance measurement unit comprises a needle-guiding device according to one of the claims 1 to 12.

14. Drilling resistance measurement unit according to claim 13,
**characterized in that**
the drilling resistance measurement unit comprises a feed drive that is configured to
- be coupled in a force-transmitting way to the drive pinions (71) and/or
- transfer the scissor-type extension arm (8') from the folded state into the extended state.

15. Method for investigating the nature of wood by use of a needle-guiding device according to at least one of the claims 1 to 7,
**comprising the steps**
- providing a drilling resistance measurement unit with the needle-guiding device (2) and clamped drilling needle (1)
- transferring the scissor-type extension arm (8') of stays (81) of the scissor-type extension arm device (8) of the support device of the drilling needle (1) of the needle-guiding device (2) into an extended state, then
- placing the needle-guiding device (2) with the drilling needle (1) against an object of wood to be examined, then
- inserting the drilling needle (1) into the object of wood and, in doing so, simultaneously transferring the scissor-type extension arm (8') into the folded state.

16. Method for investigating the nature of wood by using a needle-guiding device according to at least one of the claims 8 to 12,
**comprising the steps**
- providing a drilling resistance measurement unit with the needle-guiding device (2) and clamped drilling needle (1) and guiding the drilling needle (1) in the support device, as drive device a chain drive that comprises two chains (9) as force transmitting elements, or in the support device which as drive device a belt drive that comprises two toothed belts (10) as force transmitting elements,
- positioning the needle holder (4) in the rear position at the drive device,
- placing the needle-guiding device (2) with the drilling needle (1) against an object of wood to be examined, then inserting the drilling needle (1) into the object of wood, in doing so simultaneously
- transferring the needle holder (4) into the forward position.

## Revendications

1. Dispositif guide-aiguille (2) pour un instrument de mesure de la résistance de perçage prédéfini, qui est conçu pour guider une aiguille de perçage (1) prédéfinie et être couplé de manière opérationnelle à un entraînement de l'instrument de mesure de la résistance de perçage,
le dispositif guide-aiguille (2) présentant un dispositif de support et un porte-aiguille (4) et
- le porte-aiguille (4) présentant un mandrin qui est conçu pour recevoir l'aiguille de perçage (1) prédéfinie et
- le dispositif de support s'étendant dans le sens longitudinal de l'aiguille de perçage (1) prédéfinie,
**caractérisé en ce que**
le dispositif de support présente au moins un dispositif à grille articulée (8) avec une grille articulée (8') composée d'entretoises (81).

2. Dispositif guide-aiguille (2) selon la revendication 1,
**caractérisé en ce que**
la grille articulée (8') peut être transférée dans un état rabattu et dans un état déployé et serre un niveau E,
deux entretoises (81) se croisant étant respectivement reliées de façon articulée au moyen d'une structure de support (86) avec articulation (82),
et les structures de support (86) s'étendant normalement vers le niveau E serré par la grille articulée et
chaque structure de support (86) présentant un orifice de passage (85) comme prise pour l'aiguille de perçage (1) prédéfinie et les orifices de passage (85) de toutes les structures de support (86) étant alignés entre eux et avec un orifice (41) du porte-aiguille (4).

3. Dispositif guide-aiguille (2) selon la revendication 2,
**caractérisé en ce que**
la grille articulée (8') présente, à l'état déployé, une longueur qui est sélectionnée de manière à ce que l'aiguille de perçage (1) prédéfinie ne s'étende pas au-delà de la grille articulée (8') dans une position adoptée dans le mandrin,
et **en ce que** la grille articulée (8') présente, à l'état rabattu, une longueur qui est sélectionnée de manière à ce que l'aiguille de perçage (1) prédéfinie dépasse d'au moins la moitié de sa longueur au-delà de la grille articulée (8').

4. Dispositif guide-aiguille (2) selon la revendication 2 ou 3,
**caractérisé en ce que**
un corps moulé (87) est présent au moins à une extrémité au moins pour la majorité des structures de support (86),
les corps moulés (87) qui se trouvent sur le même côté du niveau E, affleurant les uns aux autres quand la grille articulée (8') est à l'état rabattu.

5. Dispositif guide-aiguille (2) selon au moins une des revendications 2 à 4,
**caractérisé en ce que**
la structure de support (86) est fixée, avec sa section présentant l'orifice de passage (85), directement sur l'une des entretoises (81) et la section présentant l'articulation (82) vient se loger à l'intérieur des entretoises.

6. Dispositif guide-aiguille (2) selon au moins une des revendications 2 à 5,
**caractérisé en ce que**
la structure de support (86) avec articulation (82) est en deux ou plusieurs parties et les sections sont reliables entre elles par vissage,
de préférence une première section formant filetage de la structure de support (86) étant un manchon auquel est reliée l'une des deux entretoises (81) se croisant et une deuxième section formant filetage étant une goupille à laquelle est reliée la deuxième entretoise (81), la goupille étant en prise par rotation avec le manchon.

7. Dispositif guide-aiguille (2) selon au moins une des revendications 1 à 6,
**caractérisé en ce que**
le dispositif à grille articulée (8)
- est en liaison active avec un entraînement d'avance de l'instrument de mesure de la résistance de perçage prédéfini, la grille articulée (8') pouvant être transférée de l'état rabattu à l'état déployé par l'entraînement d'avance et/ou présente deux grilles articulées (8'), dont les niveaux E sont disposés parallèlement les uns aux autres à l'état déployé, une articulation (82) d'une grille articulée (8') étant respectivement reliée à l'articulation (82) correspondante de l'autre grille articulée (8') par une structure de support (86) commune,
de préférence les grilles articulées (8') étant disposées de manière à ce que les orifices de passage (85) des structures de support (86) se trouvent entre les deux grilles articulées (8').

8. Dispositif guide-aiguille (2) pour un instrument de mesure de la résistance de perçage prédéfini, qui est conçu pour guider une aiguille de perçage (1) prédéfinie et être couplé de manière opérationnelle à un entraînement de l'instrument de mesure de la résistance de perçage,
le dispositif guide-aiguille (2) présentant un dispositif de support et un porte-aiguille (4) et
- le porte-aiguille (4) présentant un mandrin qui est conçu pour recevoir l'aiguille de perçage (1) prédéfinie et
- le dispositif de support s'étendant dans le sens longitudinal de l'aiguille de perçage (1) prédéfinie,
**caractérisé en ce que**
le dispositif de support est un dispositif d'entraînement qui présente deux éléments de transmission de force juxtaposés en parallèle et en interaction.

9. Dispositif guide-aiguille (2) selon la revendication 8,
**caractérisé en ce que**
- le dispositif d'entraînement est formé par un entraînement à chaînes qui présente deux chaînes (9) comme éléments de transmission de force ou
- le dispositif d'entraînement est formé par un entraînement à courroies qui présenté deux courroies dentées (10) comme éléments de transmission de force.

10. Dispositif guide-aiguille (2) selon la revendication 9,
**caractérisé en ce que**
les deux chaînes (9) ou deux courroies dentées (10) sont serrées respectivement à une extrémité autour d'un pignon (72) et à l'autre extrémité autour d'un pignon moteur (71),
- les pignons moteurs (71) étant conçus pour être entraînés par un entraînement d'avance de l'instrument de mesure de la résistance de perçage prédéfini et
- le porte-aiguille (4) étant fixé sur le dispositif d'entraînement et pouvant être transféré par le dispositif d'entraînement depuis une position arrière dans laquelle le porte-aiguille (4) est positionné à l'extrémité du dispositif d'entraînement qui est dans une position d'utilisation côté instrument de mesure de la résistance de perçage, dans une position avant dans laquelle le porte-aiguille (4) est positionné à l'autre extrémité du dispositif d'entraînement et
- les éléments de transmission de force présentant des éléments de guidage et un élément de guidage d'un élément de transmission de force respective étant en liaison active avec un élément de guidage de l'autre élément de transmission de force et formant une bordure pour l'aiguille de perçage (1) prédéfinie.

11. Dispositif guide-aiguille (2) selon la revendication 8 ou 9,
**caractérisé en ce que**
les deux chaînes (9) présentent des maillons (91) qui présentent des boulons (93) et des éclisses (92), la bordure pour l'aiguille de perçage (1) prédéfinie étant formée par les boulons (93) des maillons (91), les boulons (93) présentant une courbure intérieure qui correspond à une forme négative de l'aiguille de perçage (1),
et de préférence
les deux chaînes (9) étant juxtaposées de manière à ce que les éclisses (92) des maillons (91) s'engrènent.

12. Dispositif guide-aiguille (2) selon la revendication 9,
**caractérisé en ce que**
les courroies dentées (10) de l'entraînement à courroies présentent, sur le côté non denté, des crochets (11), les crochets (11) d'une courroie dentée (10) s'engrenant dans les crochets de l'autre courroie dentée (10) et les crochets (11) présentant un évidement (11') détourné de la courroie, se chevauchant dans une position en prise et formant un passage rond comme bordure pour l'aiguille de perçage (1) prédéfinie.

13. Instrument de mesure de la résistance de perçage pour l'analyse de propriétés du bois,
**caractérisé en ce que**
l'instrument de mesure de la résistance de perçage présente un dispositif guide-aiguille (2) selon une des revendications 1 à 12.

14. Instrument de mesure de la résistance de perçage selon la revendication 13,
**caractérisé en ce que**
l'instrument de mesure de la résistance de perçage présente un entraînement d'avance qui est conçu
- pour être couplé aux pignons moteurs (71) par transmission de force et/ou
- pour transférer la grille articulée (8') de l'état rabattu à l'état déployé.

15. Procédé pour l'analyse de propriétés du bois avec l'utilisation d'un dispositif guide-aiguille selon au moins une des revendications 1 à 7,
**comprenant les étapes**
- mise à disposition d'un instrument de mesure de la résistance de perçage avec le dispositif guide-aiguille (2) et une aiguille de perçage (1) serrée
- transfert de la grille articulée (8') composée d'entretoises (81) du dispositif à grille articulée (8) du dispositif de support de l'aiguille de perçage (1) du dispositif guide-aiguille (2) dans un état déployé, puis
- fixation du dispositif guide-aiguille (2) avec l'aiguille de perçage (1) sur un objet en bois à analyser, puis
- introduction de l'aiguille de perçage (1) dans l'objet en bois et, en même temps, transfert de la grille articulée (8') dans l'état rabattu.

16. Procédé pour l'analyse de propriétés du bois avec l'utilisation d'un dispositif guide-aiguille selon au moins une des revendications 8 à 12,
**comprenant les étapes**
- mise à disposition d'un instrument de mesure de la résistance de perçage avec le dispositif guide-aiguille (2) et une aiguille de perçage (1) serrée et guidage de l'aiguille de perçage (1) dans le dispositif de support présentant, comme dispositif d'entraînement, un entraînement à chaînes qui présente deux chaînes (9) comme éléments de transmission de force ou dans le dispositif de support présentant, comme dispositif d'entraînement, un entraînement à courroies qui présente deux courroies dentées (10) comme éléments de transmission de force,
- positionnement du porte-aiguille (4) dans la position arrière sur le dispositif d'entraînement,
- fixation du dispositif guide-aiguille (2) avec l'aiguille de perçage (1) sur un objet en bois à analyser, puis introduction de l'aiguille de perçage (1) dans l'objet en bois et, en même temps,
- transfert du porte-aiguille (4) dans la position avant.
